# FASCICULE DE BREVET EUROPEEN

(11) **EP 2 331 496 B1**
(45) Date de publication et mention de la délivrance du brevet: **01.06.2016**
(21) Numéro de dépôt: 09740375.2
(22) Date de dépôt: 24.07.2009
(51) Int. Cl.: A61Q 19/08, A61K 8/44

(54) **UTILISATION D'UN N-ACYL AMINO ACIDE CHOISI PARMI LE N-PALMITOYL ALANINE, LE N-PALMITOYL GLYCINE ET LE N-PALMITOYL ISOLEUCINE, COMME ACTIF REGULATEUR DU PROFIL GENETIQUE DES FIBROBLASTES REPLICATIFS SENESCENTS DU DERME DE LA PEAU HUMAINE**
VERWENDUNG EINER AUS N-PALMITOYLALANIN, N-PALMITOYLGLYCIN UND N-PALMITOYLISOLEUCIN AUSGEWÄHLTEN N-ACYLAMINOSÄURE ALS MITTEL ZUR REGULIERUNG DES GENETISCHEN PROFILS SENESZENTER REPLIZIERENDER FIBROBLASTEN DER HUMANEN HAUT
USE OF AN N-ACYL AMINO ACID SELECTED FROM AMONG N-PALMITOYL ALANINE, N-PALMITOYL GLYCINE, AND N-PALMITOYL ISOLEUCINE AS A REGULATING ACTIVE AGENT OF THE GENETIC PROFILE OF SENESCENT REPLICATING FIBROBLASTS OF THE HUMAN SKIN DERMIS

(30) Priorité: 29.08.2008 FR 0855792
(43) Date de publication de la demande: 15.06.2011
(73) Titulaire: Societe D'Exploitation De Produits Pour Les Industries Chimiques Seppic, 75007 Paris (FR)
(72) Inventeur: STOLTZ, Corinne, F-94320 Thiais (FR); DUMONT, Sandy, F-81200 Caucalieres (FR); TAILLEBOIS, Cécile, 81990 Salies (FR)
(74) Mandataire: Conan, Philippe Claude
(86) Numéro de dépôt international: PCT/FR2009/051489
(87) Numéro de publication internationale: WO 2010/023390

(56) Documents cités:
- EP-A1- 0 552 405
- WO-A1-90/14429
- FR-A- 2 192 795
- US-A1- 2007 048 396
- YATAO YU ET AL: "Synthesis and characterization of silica nanotubes with radially oriented mesopores", ADVANCED FUNCTIONAL MATERIALS, WILEY - V C H VERLAG GMBH & CO. KGAA, DE, vol. 18, no. 4, 22 February 2008 (2008-02-22), pages 541-550, XP001510813, ISSN: 1616-301X, DOI: 10.1002/ADFM.200700593
- KATAOKA EIKO ET AL: "Studies on Utilization of acylamino acids. II. Effect of N-palmitoyl amino acids on the growth of lactobacillaceae", TOKYO NOGYO DAIGAKU NOGAKU SHUHO - JOURNAL OF AGRICULTURALSCIENCE, TOKYO NOGYO DAIGAKU, TOKYO NOGYO GAIGAKU, TOKYO, JP, vol. 19, no. 2, 1 January 1974 (1974-01-01), pages 102-111, XP008160429, ISSN: 0375-9202

## Description

La présente invention se rapporte au domaine des principes actifs cosmétiques et pharmaceutiques ainsi qu'aux compositions topiques en comportant.

L'augmentation de l'espérance de vie dans les pays développés s'est constamment accompagnée par un désir irrépressible de "rajeunissement" qui s'est accentué depuis une trentaine d'années et qui se traduit par des tentatives pour retarder sinon voir disparaître, l'apparition de signes de vieillissement de la peau tels que notamment les rides. Cette évolution comportementale s'est accompagnée par un développement de la recherche scientifique dédiée à la peau ainsi que depuis une dizaine d'années par une croissance rapide du marché des produits cosmétiques dits "anti-âge".

La présente invention s'inscrit dans ce cadre de la recherche de nouvelles molécules ou compositions qui, appliqués sur la peau, produisent par leur activité propre cet effet "anti-âge".

Le mécanisme du vieillissement de la peau peut être expliqué comme suit :
La peau est composée de deux tissus majoritaires : le derme et l'épiderme, plus superficiel, entre lesquels s'intercale une zone de jonction, la jonction dermo-épidermique.
   - Le derme est un tissu conjonctif dense et fibro-élastique, dont la production et le remodelage éventuel sont essentiellement assurés par les fibroblastes et qui est constitué de la matrice extracellulaire (MEC) composée de molécules fibreuses (collagéniques et élastiques), responsables des qualités esthétiques de la peau, ainsi que de la substance fondamentale. Les collagènes représentent 98% du poids sec du derme. La substance fondamentale est composée :
      - de macromolécules qui comblent les espaces entre les cellules et les fibres ;
      - de glycoprotéines de structure, qui permettent la connexion des cellules à la MEC et qui sont essentiellement localisées sous la jonction dermo-épidermique ;
      - de protéoglycanes, dont les glycosaminoglycanes (GAG), qui se présentent sous une forme liée à une protéine (liaison covalente), ou le biglycane.
   - L'épiderme est essentiellement composé de kératinocytes. Ces cellules prolifèrent dans l'assise basale puis se différencient progressivement pour donner les différentes couches de l'épiderme en migrant depuis la profondeur vers la surface, où elles desquament.
   - La jonction dermo-épidermique (JDE) est la zone de jonction entre les kératinocytes basaux de l'épiderme et le derme. Elle se caractérise par la présence d'une membrane basale, dans laquelle la MEC forme un treillis mince et solide. Elle est constituée de cinq composants majeurs, *i.e.* le collagène de type IV, les laminines, l'héparan sulfate, le nidogène (ou entactine) et la fibronectine.

L'intégrité de la MEC et de la JDE ainsi que la différenciation épidermique dépendent notamment de la présence d'enzymes responsables de la dégradation de nombreux composants de la MEC, les MMP (matrix metalloproteinases en langue anglaise).

De nombreux facteurs exogènes et endogènes, dont le stress oxydant, concourent à l'accentuation des processus inflammatoires au cours du vieillissement, comme la production d'interleukine-6 (IL-6), (Ramasamy et al. 2005 ; Papanicolaou et al. 2006 ; Vaeto et al. 2007). L'une, l'autre ou plusieurs des molécules de la JDE, listées ci-dessus, sont altérées par les radicaux libres au cours du vieillissement cutané.

Il existe aujourd'hui de nombreux principes actifs dits "anti-âge" ou "antirides". Plusieurs d'entre eux sont capables de réguler le phénotype et/ou les propriétés de fibroblastes humains normaux. Par exemple, de nombreux principes actifs cosmétiques stimulent la production fibroblastique de collagène. Ainsi, parmi les LAA utilisés dans des applications cosmétiques, certains présentent des propriétés biologiques anti-âge, tels que le dipalmitoyl hydroxyproline (SEPILIFT™ DPHP).

La demande de brevet américain publiée sous le numéro US 2006/024375 divulgue que l'incorporation d'un mélange de N-palmitoyl proline, d'acide N-palmitoyl gutamique et de N-palmitoyl sarcosine dans un fond de teint induit un effet protecteur de la peau et un effet anti-âge supérieur notamment à cause de sa propriété anti-inflammatoire sur la peau et de son action comme antagoniste de la substance P.

La demande de brevet américain publiée sous le numéro US 2007/0048396 divulgue les propriétés anti-inflammatoires sur la peau d'un mélange de N-cocoyl amino acides, d'aspartate de magnésium et d'aspartate de potassium.

En revanche, peu de principes actifs "anti-âge" sont connus pour leur capacité à induire une réduction de la composante inflammatoire, en particulier la diminution du niveau d'expression de l'IL-6. En effet, si certains antioxydants sont capables de réduire les productions d'IL-6 associées au vieillissement (Walston et al. 2006; Omoigui, 2007), de telles actions ne sont pas forcément corrélées à une capacité à réguler la production de MEC ou à la protéger. Au contraire, certains principes actifs, tels que le rétinol sont décrits comme inducteurs d'une irritation (Kim et al. 2003). En outre, certaines molécules pharmaceutiques anti-inflammatoires induisent une diminution de la production des composants de la MEC tels que les fibres de collagène (Miltyk et al. 1996). Il n'existe donc pas de corrélation évidente entre les propriétés anti-inflammatoires des principes actifs d'une part et leur efficacité anti-âge d'autre part.

Différents modèles expérimentaux ont été décrits afin de visualiser les effets du vieillissement cutané puis de les corriger. Ainsi, dans le cas des cultures de fibroblastes en monocouche, différents stimuli peuvent conduire à un vieillissement accéléré *in vitro*.
- Par exemple, il est possible de soumettre ces cellules à une irradiation aux rayonnements ultraviolet afin de mimer le photo-vieillissement et/ou un stress oxydant (Ma et al. 2001 ; Kawanishi et Oikawa, 2004 ; Chen et al. 2007).
- Le vieillissement peut également être reproduit *in vitro* en cultivant et en étudiant des cellules de donneurs âgés [Albini et al. (1988) ; Miyoshi et al. (2006)].
- Enfin, d'après le modèle de Hayflick, la sénescence peut être reproduite en cultivant les fibroblastes de manière à leur faire réaliser un grand nombre de doublements de populations (Bradley, Hayflick et Schimke, 1976 ; Albini et al.. 1988). Ce processus est communément dénommé "sénescence réplicative".

Ces modèles de vieillissements expérimentaux sur des cultures primaires peuvent donc être utilisés pour évaluer la capacité d'un principe actif cosmétique à corriger un défaut et/ou une exagération d'un phénotype ou d'une fonction biologique induit(s) par le vieillissement cutané. Ainsi, de nombreux principes actifs limitent la production protéique de MMP au niveau des fibroblastes irradiés par des UV. D'autres sont capables de restaurer partiellement l'activité d'un gène ou d'une protéine dans des fibroblastes de passage tardif.

Cependant, certaines études sont menées sur des lignées cellulaires et non sur des cultures primaires.

Or, les lignées cellulaires se caractérisent par leur capacité à réaliser des doublements de population de manière quasi-illimitée. Ce processus correspond à l'immortalisation (Goldstein, 1990 ; Rhim, 1991 ; Macieira-Coelho, 2000). Ainsi, il est communément admis que les lignées présentent certaines différences avec les cultures primaires équivalentes. Elles ne reflètent donc pas la réalité physiologique, en particulier dans le contexte du vieillissement. Par ailleurs, dans le cas des cultures primaires de fibroblastes humains, la plupart des études sont réalisées à l'aide de cellules provenant de prépuces. Or, ces dernières présentent des caractéristiques particulières par rapport aux fibroblastes humains issus de derme adulte (Jelaska et Korn, 1998 ; Khorramizadeh et al. 1999 ; Saguet et al. 2006). En particulier, il est communément admis que ces fibroblastes présentent des capacités de prolifération accrues. Leur sensibilité au stress oxydant serait également différente (Saguet et al. 2006). Il est donc plus pertinent d'utiliser des cellules de même origine que celles qui seront finalement ciblées par le principe actif anti-âge, c'est-à-dire les cellules de derme humain adulte. Or, à notre connaissance, aucun N-acyl amino acide n'a été décrit comme capable de réguler le profil génétique de fibroblastes réplicatifs sénescents issus du derme humain adulte de manière à réduire l'inflammation tout en favorisant la production ou la protection de la MEC.

La présente demande divulgue l'utilisation d'un N-acyl amino acide choisi parmi le N-palmitoyl alanine, le N-palmitoyl glycine, le N-palmitoyl isoleucine et le N-cocoyl alanine, pour la mise en oeuvre d'une méthode thérapeutique, destinée à retarder l'apparition des rides et/ou à ralentir le vieillissement cellulaire de la peau du corps humain.

Par régulateur du profil génétique des fibroblastes réplicatifs sénescents du derme de la peau humaine, on désigne plus particulièrement que le N-acyl amino acide tel que défini ci-dessus, présente l'une, l'autre, plusieurs ou l'ensemble des propriétés suivantes :
- il induit une diminution du niveau d'expression du gène codant la cytokine pro-inflammatoire IL-6 ;
- il induit une diminution du niveau d'expression du gène codant la MMP-1, une métallo protéinase impliquée dans la dégradation de la matrice extracellulaire ;
- il induit une diminution du niveau d'expression du gène codant une protéine favorisant l'inflammation, la Serpine 2B ou PAI (Plasminogen activator inhibitor)-2 (Kessler-Becker et al. 2004) ;
- il induit une augmentation du niveau d'expression du gène codant le dermique dermatopontine (DPT), un des composants de la matrice extracellulaire (Okamoto et Fujiwara, 2006);
- il induit une augmentation du niveau d'expression du gène codant le collagène de type I (COL1A1), un des composants de la matrice extracellulaire ;
- il induit une augmentation du niveau d'expression du gène codant le biglycane (BGN), un des composants de la matrice extracellulaire ;
- il induit une augmentation du niveau d'expression du gène codant le nidogène, un des composants de la jonction dermo-épidermique (Aumailley et al. 1984) ;
- il induit une augmentation du niveau d'expression du gène codant la tenascine C (TNC), un des composants de la jonction dermo-épidermique (Marionnet et al. 2006) et également impliquée dans la contraction des fibres de collagène (Tamaoki et al. 2005);
- Il induit une augmentation du niveau d'expression du gène codant la pleiotrophine (PTN), une protéine impliquée dans les interactions entre les fibroblastes et les kératinocytes (Florin et al. 2005) ;
- Il induit une augmentation du niveau d'expression du gène codant la protéine de choc thermique (HSP 90A), impliquée dans la régulation de l'activité du protéasome, système permettant la dégradation des protéines endommagées (Morishima et al. 2005). ;
- Il induit une augmentation du niveau d'expression du gène codant la Serpine 1H (HSP47), une protéine impliquée dans la régulation de la formation des fibres de collagène (Kuroda et Tajima, 2004) ;
- il induit une augmentation du niveau d'expression du gène codant la vimentine, un des composants du cytosquelette des fibroblastes (Rappersberger et al. 1990).

Le N-acyl amino acide mis en oeuvre dans les utilisations telle que définies ci-dessus, peut être sous forme d'acide libre ou sous forme partiellement ou totalement salifiée. Lorsqu'il est sous forme salifiée, il s'agit notamment de sels alcalins tels que les sels de sodium, de potassium ou de lithium, de sels alcalino-terreux tels que les sels de calcium, de magnésium ou de strontium ; de sel d'ammonium ou de sel d'un amino-alcool comme le sel de (2-hydroxy éthyl) ammonium. Il peut aussi s'agir de sels métalliques tels que les sels divalents de zinc ou de manganèse, les sels trivalents de fer, de lanthane, de cérium ou d'aluminium. De façon générale, le taux de salification dudit N-acyl amino acide, dépendra en autre de son pK_{A} et de la concentration en sels de la composition dans laquelle il est incorporé.

Selon une autre variante particulière de la présente demande on utilise un seul des N-acyl amino acides définis précédemment, dans la composition contenant le milieu cosmétiquement acceptable.

Selon une autre variante particulière de la présente demande on utilise un mélange de N-acyl amino acides choisis par ceux définis précédemment.

Par N-cocoyl alanine, on désigne un mélange de N-acyl alanine obtenu par acylation de l'alanine avec les dérivés activés d'acides gras issus de l'huile de coprah.

Le N-acyl amino acide tel que défini précédemment, est généralement obtenu par N-acylation des acides aminés correspondants ou de leurs sels.

Lorsqu'il s'agit d'un mélange de N-acyl amino acides il est par exemple obtenu par N-acylation du mélange d'acides aminés résultant de l'hydrolyse totale de protéines de toutes origines.

L'invention a pour objet, un procédé de traitement non thérapeutique du corps humain destiné à retarder l'apparition des rides et/ou ralentir le vieillissement cellulaire, caractérisé en ce que l'on y applique une composition contenant un milieu cosmétiquement acceptable et une quantité efficace d'au moins un N-acyl amino acide choisi parmi le N-palmitoyl glycine et le N-palmitoyl isoleucine.

Selon un autre aspect particulier, l'invention a pour objet, un procédé tel que défini précédemment pour laquelle le N-acyl amino acide est le N-palmitoyl glycine.

Selon un autre aspect particulier, l'invention a pour objet, un procédé tel que défini précédemment pour laquelle le N-acyl amino acide est le N-palmitoyl isoleucine.

Dans les utilisations et dans le procédé tels que définis ci-dessus, le N-acyl amino acide est généralement mis en oeuvre dans une composition destinée à une application topique sur la peau et les muqueuses. Ladite composition destinée à une application topique en comprend entre 0,01 % et 10 % massique, plus particulièrement entre 0,1 % à 5 % massique, et tout particulièrement entre 1 % et 5 % massique dudit ou desdits N-acyl amino acides. Ledit traitement ou les dites utilisations sont mises en oeuvre par application de ladite composition destinée à une application topique, sur la surface de la peau ou de la muqueuse à traiter.

Selon un autre aspect, la présente demande divulgue une composition cosmétique "anti-âge" caractérisée en ce qu'elle comprend de 0,01 % à 10 % massique, plus particulièrement de 0,1 % 5 % massique et tout particulièrement de 1 % à 5 % massique d'un N-acyl amino acide choisi parmi le N-palmitoyl glycine, le N-palmitoyl alanine, le N-palmitoyl isoleucine et le N-cocoyl alanine.

La composition mises en oeuvre dans ledit procédé ou dans lesdites utilisations, se présentent généralement sous forme de solutions aqueuses ou hydro-alcooliques diluées, sous forme d'émulsions simples ou multiples, telles que les émulsions eau dans huile (E/H), huile dans eau (H/E) ou eau dans huile dans eau (E/H/E), dans lesquelles l'huile est de nature végétale ou minérale, ou sous forme de poudre. Elles peuvent aussi être dispersées ou imprégnées sur du textile ou sur des matériaux non tissés qu'il s'agisse de lingettes, de serviettes en papier ou de vêtements.

De façon générale, les N-acyl amino acides tels que définis précédemment, sont associés à de nombreux types d'adjuvants ou de principes actifs utilisés dans les formulations cosmétiques, qu'il s'agisse, de corps gras, de solvants organiques, d'épaississants, de gélifiants, d'adoucissants, d'antioxydants, d'opacifiants, de stabilisants, de moussants, de parfums, d'émulsionnants, ioniques ou non, de charges, de séquestrants, de chélateurs, de conservateurs, de filtres chimiques ou de filtres minéraux, d'huiles essentielles, de matières colorantes, de pigments, d'actifs hydrophiles ou lipophiles, d'humectants, par exemple la glycérine, de conservateurs, de colorants, de parfums, d'actifs cosmétiques, de filtres solaires minéraux ou organiques, de charges minérales comme les oxydes de fer, oxydes de titane et le talc, de charges synthétiques comme les nylons et les poly(méthacrylate de méthyle) réticulés ou non, d'élastomères silicone, de séricites ou d'extraits de plantes ou encore de vésicules lipidiques ou tout autre ingrédient habituellement utilisé en cosmétique.

Comme exemples d'huiles que l'on peut associer au N-acyl amino acide tel que défini précédemment, on peut citer, les huiles minérales telles que l'huile de paraffine, l'huile de vaseline, les isoparaffines ou les huiles blanches minérales, les huiles d'origine animale, telles que le squalène ou le squalane, les huiles végétales, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ; les huiles végétales éthoxylées ; les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadécane, l'isododécane, les huiles perfluorées et les huiles de silicone. Parmi ces dernières, on peut plus particulièrement citer les diméthylpolysiloxanes, méthylphénylpolysiloxanes, les silicones modifiées par des aminés, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Comme autre matière grasse que l'on peut associer à cet actif, on peut citer les alcools gras ou les acides gras.

Parmi les polymères épaississants et/ou émulsionnant pouvant être associés dans les préparations cosmétiques comprenant le N-acyl amino acide tel que défini précédemment, il y a par exemple, les homopolymères ou copolymères de l'acide acrylique ou de dérivés de l'acide acrylique, les homopolymères ou copolymères de l'acrylamide, les homopolymères ou copolymères de dérivés de l'acrylamide, les homopolymères ou copolymères de l'acide acrylamidométhyl propanesulfonique, de monomère vinylique, de chlorure de triméthylaminoéthylacrylate, les hydrocolloïdes d'origine végétale ou biosynthétique, par exemple la gomme de xanthane, la gomme de karaya, les carraghénates, les alginates ; les silicates ; la cellulose et ses dérivés ; l'amidon et ses dérivés hydrophiles ; les polyuréthanes.

Parmi les polymères de type polyélectrolytes, pouvant être mis en jeu dans la production d'une phase aqueuse gélifiée apte à être utilisée dans la préparation d'émulsions E/H contenant le N-acyl amino acide objet de la présente invention, il y a par exemple, les copolymères de l'acide acrylique et de l'acide-2-méthyl-[(1-oxo-2-propényl)amino]1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone. De tels polymères sont commercialisés respectivement sous les appellations SIMULGEL™ EG, SEPIGEL™ 305, SIMULGEL™ NS, SIMULGEL™ 800 et SIMULGEL™ A par la demanderesse.

Parmi les cires utilisables dans la présente invention, on peut citer par exemple la cire d'abeille ; la cire de carnauba ; la cire de candelilla ; la cire d'ouricoury ; la cire du Japon ; la cire de fibre de liège ou de canne à sucre ; les cires de paraffines ; les cires de lignite ; les cires microcristallines ; la cire de lanoline ; l'ozokérite ; la cire de polyéthylène ; les huiles hydrogénées ; les cires de silicone ; les cires végétales ; les alcools gras et les acides gras solides à température ambiante ; les glycérides solides à température ambiante.

Parmi les émulsionnants utilisables dans la présente invention, on peut citer par exemple les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435 et 2 804 432.

Comme exemples de principe actif que l'on peut associer au N-acyl amino acide tel que défini précédemment, on peut citer les composés ayant une action éclaircissante ou dépigmentante tels que par exemple l'arbutine, l'acide kojique, l'hydroquinone, l'acide ellagique, la vitamine C, le magnésium ascorbyl phosphate les extraits de polyphénols, les extraits de raisin, les extraits de pin, les extraits de vin, les extraits d'olives, les extraits de mare, les protéines N-acylées, les peptides N-acylés, les acides aminés N-acylés, les hydrolysâts partiels de protéines N-acylés, les acides aminés, les peptides, les hydrolysâts totaux de protéines, les hydrolysâts partiels de protéines, les polyols (par exemple, la glycérine ou le butylène glycol), l'urée, l'acide pyrrolidonecarboxylique ou les dérivés de cet acide, l'acide glycyrrhétinique, l'alpha-bisabolol, les sucres ou les dérivés des sucres, les polysaccharides ou leurs dérivés, les hydroxyacides par exemple l'acide lactique, les vitamines, les dérivés de vitamines comme le Rétinol, la vitamine E et ses dérivés, les minéraux, les enzymes, les co-enzymes, comme, le Coenzyme Q10, les hormones ou "hormone like", les extraits de soja par exemple, la Raffermine™, les extraits de blé par exemple la Tensine™ ou la Gliadine™, les extraits végétaux, tels que les extraits riches en tanins, les extraits riches en isoflavones ou les extraits riches en terpènes, les extraits d'algues d'eau douce ou marines, les cires essentielles, les extraits bactériens, les minéraux, les lipides en général, les lipides tels que les céramides ou les phospholipides, les actifs ayant une action amincissante comme la caféine ou ses dérivés, les actifs ayant une activité antimicrobienne ou une action purifiante vis-à-vis des peaux grasses tels que le LIPACIDE™ PVB, les actifs ayant une propriété énergisante ou stimulante comme le SEPITONIC™ M3 ou le Physiogényl™ le panthénol et ses dérivés comme le SEPICAP™ MP, les actifs anti-âge comme le SEPILIFT™ DPHP, le LIPACIDE™ PVB, le SEPIVINOL™, le SEPIVITAL™, les actifs hydratants comme le SEPICALM™ S, le SEPICALM™ VG et le SEPILIFT™ DPHP, les actifs anti-âge " anti-photo vieillissement ", les actifs protecteurs de l'intégrité de la jonction dermo-épidermique, les actifs augmentant la synthèse des composants de la matrice extracellulaire, les actifs ayant une activité amincissante, raffermissante ou drainante comme la caféine, la théophylline, l'AMPc, le thé vert, la sauge, le ginko biloba, le lierre, le marron d'inde, le bambou, le ruscus, le petit houx, la centella asiatica, la bruyère, l'ulmaine, le fucus, le romarin, la saule , des actifs créant une sensation de « chauffe » sur la peau comme les activateurs de la microcirculation cutanée (exemple des nicotinates) ou des produits créant une sensation de « fraîcheur » sur la peau (exemple du menthol et des dérivés).

Comme filtre solaire que l'on peut incorporer dans la composition selon l'invention, on peut citer tous ceux figurant dans la directive cosmétique 76/768/CEE modifiée annexe VII.

L'étude expérimentale suivante illustre l'invention sans toutefois la limiter. Elle met en que les N-acyl amino acides tels que définis précédemment, régulent le profil génétique de fibroblastes réplicatifs sénescents issus du derme humain adulte et favorisent ainsi la production ou la protection de la matrice extracellulaire, tout en réduisant l'inflammation de la peau, et permettent ainsi de retarder des rides. Cette étude montre aussi que les dits N-acyl amino acides codent des protéines favorisant les interactions entre les fibroblastes et la MEC ainsi que les interactions entre les fibroblastes et les kératinocytes. L'ensemble de ces variations génétiques renforce leur propriété "anti-âge".

### Mise en évidence de l'efficacité "restauratrice" de N-acyl amino acides selon l'invention vis-à-vis du proril génétique des fibroblastes humains dermiques réplicatifs sénescents :

### Protocole

Des fibroblastes humains normaux issus de derme adulte et vieillis de manière accélérée (16 passages en culture) ou non (8 passages en culture) ont été cultivés en présence ou non de chacun des quatre N-acyl amino acides suivants : N-palmitoyl glycine, N-palmitoyl alanine, N-palmitoyl isoleucine et N-cocoyl alanine pendant 96 heures. A la fin de l'expérience, les cellules ont été lavées et extraites pour l'analyse génomique. Cette dernière a été réalisée par Q-PCR ("Quantitative Polymerase Chain Reaction").

### Résultats

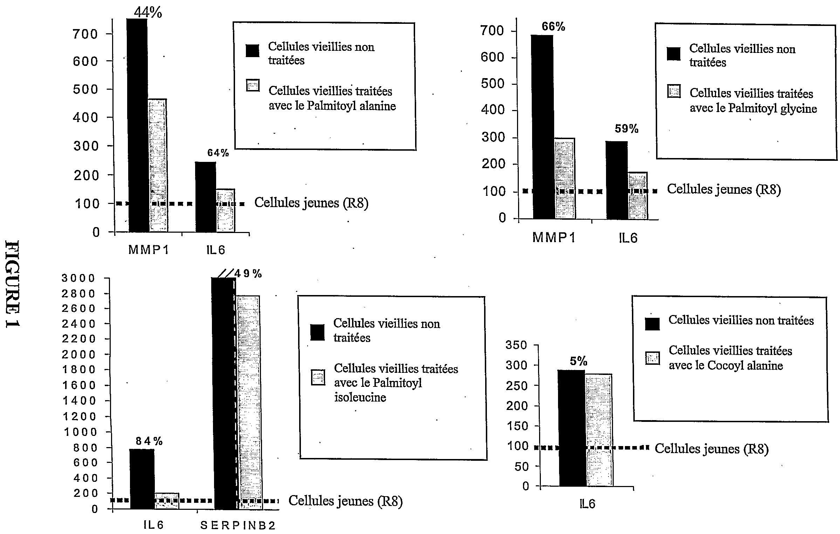

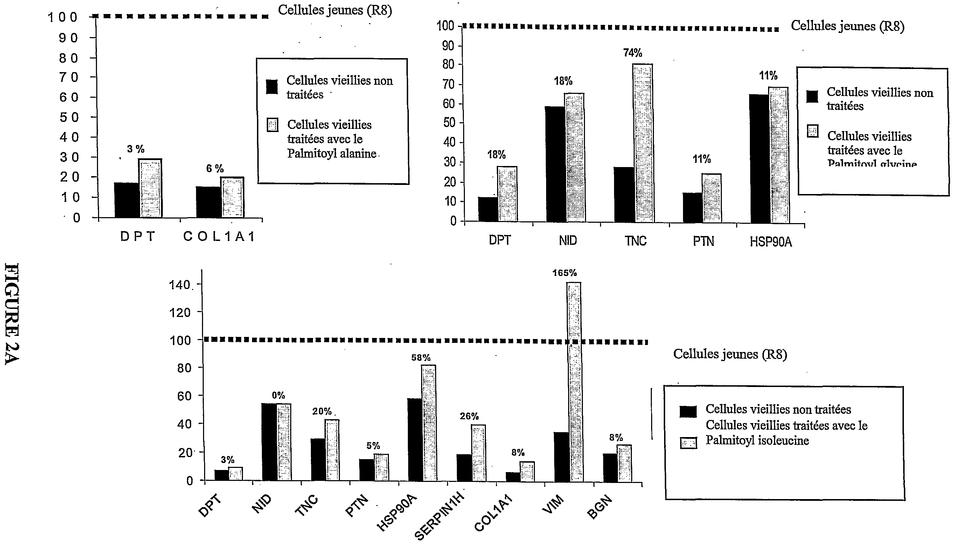

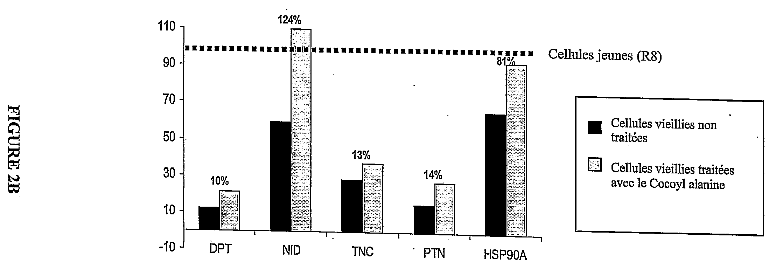

Les figures 1 et 2 montrent que les régulations géniques induites par les N-acyl amino acides testés au niveau des fibroblastes humains vieillis codent au minimum des protéines favorisant la production de matrice extracellulaire ou en limitant la dégradation et/ou d'autre part, réduisant l'inflammation cutanée.
La Figure 1 représente les diagrammes illustrant la capacité de chacun des N-acyl amino acides testés à réduire la surexpression génique associée au vieillissement des fibroblastes humains normaux.
Les Figures 2A et 2B représentent les diagrammes illustrant la capacité de chacun des N-acyl amino acides testés à compenser la diminution d'expression génique associée au vieillissement des fibroblastes humains normaux

Pour chacun des diagrammes de ces figures, les résultats sont exprimés en pourcentage de variation des niveaux relatifs d'expression génique par rapport aux cellules jeunes (100 %), les pourcentages en haut des histogrammes expriment les pourcentages de restauration et les abréviations indiquées sont conformes à la nomenclature officielle Genbank.

Ces diagrammes mettent en évidence les résultats suivants :
- Le palmitoyl alanine induit une diminution du niveau d'expression des gènes codant la cytokine pro-inflammatoire IL-6 et la métallo protéinase MMP-1, ainsi qu'une augmentation du niveau d'expression des gènes codant la dermatopontine (DPT) et le collagène de type I (COL1A1).
- Le palmitoyl glycine induit une diminution du niveau d'expression des gènes codant l'IL-6 et la MMP-1, ainsi qu'une augmentation du niveau d'expression des gènes codant la DPT, le nidogène (NID), la tenascine C (TNC), la pleiotrophine (PTN), et la protéine de choc thermique (HSP 90A).
- Le palmitoyl isoleucine induit une diminution du niveau d'expression de deux gènes codant l'IL-6, la Serpine 2B ainsi qu'une augmentation du niveau d'expression des gènes codant la DPT, le COL1A1, le biglycane (BGN), le NID, la TNC, la PTN, le HSP90A, la Serpine 1H (HSP47) et la vimentine.
- Le Cocoyl alanine induit une diminution du niveau d'expression du gène codant l'IL-6 ainsi qu'une augmentation du niveau d'expression des gènes codant la DPT, le NID, la TNC, la PTN, et l'HSP90A.

Outre leurs propriétés anti-âge, en particulier leur capacité à protéger la matrice extracellulaire ou à en promouvoir sa production, l'ensemble des N-acyl amino acides testés présentent un effet anti-inflammatoire. Cette double efficacité leur confère un avantage certain d'une part par rapport à d'autres principes actifs irritants ou d'autre part à des molécules anti-inflammatoires susceptibles d'induire une diminution de la production matricielle. Enfin, ces LAA peuvent induire d'autres régulations géniques renforçant leur efficacité anti-âge.

### Mise en évidence "in vivo" de l'efficacité "antirides" des N-acyl amino acides selon l'invention

### Protocole

Les N-acyl amino acides testés ont été formulés en gel-crème à 1% dans le couple d'émulsionnants MONTANOV™ 202, MONTANOV™ 82, l'épaississant SEPIPLUS™ 400 ainsi que le MONTANOX™ 60D, afin d'effectuer des applications topiques biquotidiennes sur le visage de sujets caucasiens âgés de 45 ans à 76 ans et présentant des rides de vieillissement cutané (environ 20 volontaires/groupe).
Un produit lipopeptidique commercial de référence a également été testé selon le même protocole expérimental.

La région anatomique choisie pour cette étude était la zone du contour de l'oeil (patte d'oie).

La durée du traitement a été de 42 jours.

L'absence d'irritation cutanée avait préalablement été confirmée par pose d'un patch de la formulation à tester pendant 48 heures.

### Résultats

Les résultats ont été exprimés en termes de surface ridée totale et de nombre de rides par rapport à un produit lipopeptidique commercial de référence.

Le N- Palmitoyl alanine a induit une diminution de 15 % de la surface ridée totale.

Le N-Palmitoyl glycine, le N-Palmitoyl isoleucine, et le N-Cocoyl alanine ont induit une diminution de respectivement 35 % ; 40 % et 33 % du nombre de rides.

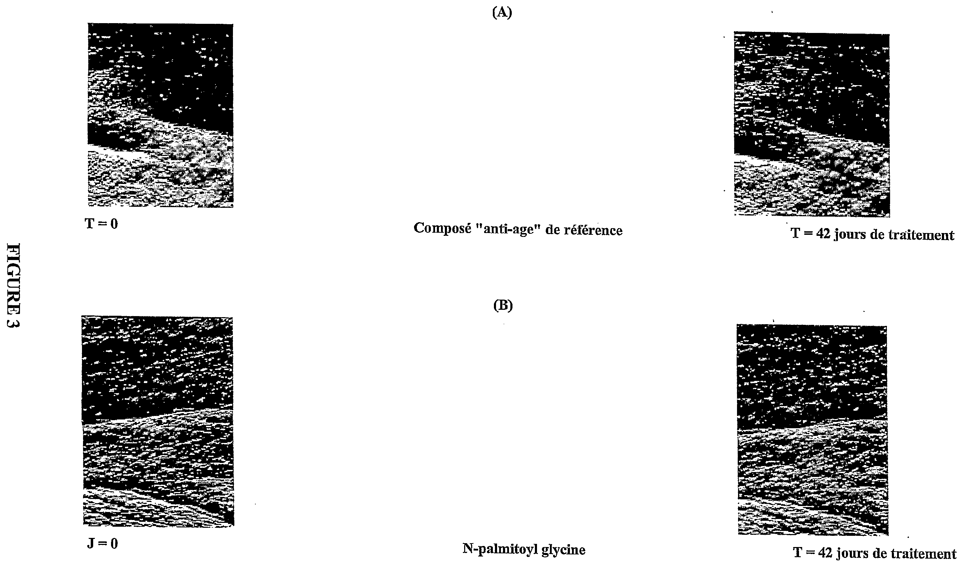

La Figure 3 rassemble les différentes photos de la zone de traitement (patte d'oie) en début de test et après 42 jours de traitement biquotidien (A) avec le lipopeptide de référence, (B) avec le N-palmitoyl glycine.

### Exemple de formulation cosmétique

| Ingrédients | % |
|---|---|
| Eau | Qsp. |
| Glycérine | 5 |
| Chlorphénesine | 0,3 |
| MONTANOV™ 202 | 2 |
| MONTANOV™ 82 | 1 |
| LANOL™ P | 1,5 |
| Squalane végétal | 7 |
| Polyisobutène | 13 |
| KETROL™ T | 0,15 |
| N-palmitoyl glycine | 1 |
| MONTANOX™ 60 DF | 0,5 |
| SEPIPLUS™ 400 | 0,8 |
| SEPICIDE™ LD | 0,7 |
| Parfum | 0,1 |
| Tri éthanolamine à 50% dans l'eau | 0,15 |

Les définitions des produits commerciaux utilisés comme ingrédients sont indiquées ci- dessous :
KETROL™ T est de la gomme de xanthane commercialisée par la société KELCO.
LANOL™ P est un additif à effet stabilisant commercialisé par la société SEPPIC.
MONTANOX™ 60 DF : Stéarate de sorbitan polyéthoxylé avec 20 moles d'oxyde d'éthylène, commercialisé par la société SEPPIC;
MONTANOV™ 82 est un agent émulsionnant à base d'alcool cétéarylique et de cocoylglucoside ;
MONTANOV™ 202 (arachidyl glucoside, alcool arachidylique + alcool béhènylique), est une composition auto-émulsionnable telle que celles décrites dans WO 98/17610, commercialisée par la société SEPPIC ;
SEPICIDE™ LD : conservateur commercialisé par la société SEPPIC ;
SEPIPLUS™ 400 : Nom INCI: polyacrylate 13 / polyisobutène / polysorbate 20

### Références bibliographiques citées dans la description.

Albini et al. 1988: Albini et al. Coll. Relat. Res. 8(1): 23-37 (1988); "Décline of fibroblast chemotaxis with age of donor and cell passage number".
Aumailley et al., 1984: Aumailley et al._J. Biol. Chem. 262(24): 11532 (1984); "The cellular interactions of laminin fragments. Cell adhesion correlates with two fragment-specific high affinity binding sites".
Bradley, Hayflick and Schimke, 1976: Bradley, Hayflick and Schimke, J. Biol. Chem. 251(12): 3521-9 (1976); "Protein degradation in human fibroblasts (WI-38). Effects of aging, viral transformations and amino acid analogs".
Chen et al., 2007: Chen et al., Methods Mol Biol.371:179-89 (2007); "Methods of cellular senescence induction using oxidative stress".
Florin et al., 2005: Florin et al., J. Cell Sci. 118(Pt 9):1981-9. (Epub 2005); "Increased keratinocyte proliferation by JUN-dependent expression of PTN and SDF-1 in fibroblasts".
Goldstein, 1990: Goldstein, Science, 249(4973):1129-33 (1990); "Replicative senescence: the human fibroblast comes of age".
Jelaska et Korn, 1998: Jelaska et Korn, J. Cell Physiol. 175(1):19-29 (1998); "Anti-Fas induces apoptosis and proliferation in human dermal fibroblasts: differences between foreskin and adult fibroblasts".
Kawanishi et Oikawa: Kawanishi et Oikawa, Ann N Y Acad. Sci. Jun; 1019: 278-84 (2004); "Mechanism of telomere shortening by oxidative stress".
Kessler-Becker et al., 2004 : Kessler-Becker et al. Exp Dermatol. 13(11):708-14 (2004): "High plasminogen activator inhibitor type 2 expression is a hallmark of scleroderma fibroblasts in vitro".
Khorramizadeh et al., 1999: Khorramizadeh et al., Mol Cell Biochem. 194(1-2):99-108 (1999); "Aging differentially modulates the expression of collagen and collagenase in dermal fibroblasts".
Kim et al., 2003: Kim et al. Toxicol. Lett. 15; 146(1): 65-73 (2003); "The mechanism of retinol-induced irritation and its application to anti-irritant development".
Kuroda et Tajima, 2004: Kuroda et Tajima, J Cutan. Pathol. 31(3):241-6 (2004); "HSP47 is a useful marker for skin fibroblasts in formalin-fixed, paraffin-embedded tissue specimens".
Ma et al., 2001: Ma et al. Clin. Exp. Dermatol. Oct; 26(7):592-9 (2001); "Chronological ageing and photoageing of the fibroblasts and the dermal connective tissue".
Macieira-Coelho, 2000: Macieira-Coelho, Prog Mol Subcell Biol.24:51-80 (2000); "Comparative biology of cell immortalization".
Marionnet et al. (2006): Marionnet et al. J Invest Dermatol. 126(5):971-9 (2006); "Interactions between fibroblasts and keratinocytes in morphogenesis of dermal epidermal junction in a model of reconstructed skin".
Miltyk et al., 1996: Miltyk et al., Pol. J. Pharmacol. 48(6): 609-13 (1996); "Inhibition of prolidase activity by non-steroid anti-inflammatory drugs in cultured human skin fibroblasts".
Miyoshi et al. 2006: Miyoshi et al. Proc. Natl. Acad. Sci. USA. Feb 7; 103(6):1727-31 (2006); "Age-dependent cell death and the role of ATP in hydrogen peroxide-induced apoptosis and necrosis";
Morishima et al. 2005: Morishima et al Biochemistry. 44(49):16333-40 (2005); "Regulation of cytochrome P450 2E1 by heat shock protein 90-dependent stabilization and CHIP-dependent proteasomal degradation".
Okamoto et Fujiwara, 2006: Okamoto et Fujiwara, Connect Tissue Res. 47(4):177-89 (2006); "Dermatopontin, a novel player in the biology of the extracellular matrix".
Omoigui, 2007: Omoigui, Immun Ageing. 4:1 (2007); "The Interleukin-6 inflammation pathway from cholesterol to aging--role of statins, bisphosphonates and plant polyphenols in aging and age-related diseases".
Papanicolaou et al., 2006: Papanicolaou et al. Ann Intern Med. 15; 128(2):127-37 (2006); "The pathophysiologic roles of interleukin-6 in human disease".
Ramasamy et al. 2005: Ramasamy et al. Glycobiology 15(7):16R-28R. (2005); "Advanced glycation end products and RAGE: a common thread in aging, diabetes, neurodegeneration, and inflammation".
Rappersberger et al. 1990: Rappersberger et al. J Invest Dermatol. 19909 4(5):700-5 (1990); "Immunogold staining of intermediate-sized filaments of the vimentin type in human skin: a postembedding immunoelectron microscopic study".
Rhim, 1991: Rhim, Yonsei Med J. 32(3):195-206 (1991); Current state-of-the-art in human cell transformation in culture.
Saguet et al. 2006: Saguet et al., Eur J Dermatol. 16(4):368-74 (2006); "Comparison of ultraviolet B-induced imbalance of antioxidant status in foreskin- and abdominal skin-derived human fibroblasts".
Tamoki et al. 2005: Tamoki et al. Am J Pathol. Jul; 167(1):71-80 (2005); "Tenascin-C regulates recruitment of myofibroblasts during tissue repair after myocardial injury".
Vaeto et al. 2007: Vaeto et al. Mech Ageing Dev. 128(1):83-91 (2007) (Epub 2006 Nov 21); "Inflammatory networks in ageing, age-related diseases and longevity".
Walston et al. 2006: Walston et al., Am J Epidemiol. 163(1):18-26 (2006) (Epub 2005 Nov 23); "Serum antioxidants, inflammation, and total mortality in older women".

## Revendications

1. Procédé de traitement non thérapeutique du corps humain destiné à retarder l'apparition des rides, **caractérisé en ce que** l'on y applique une composition contenant un milieu cosmétiquement acceptable et une quantité efficace d'au moins un N-acyl amino acide choisi parmi le N-palmitoyl glycine et le N-palmitoyl isoleucine.

2. Procédé tel que défini à la revendication 1, pour lequel le N-acyl amino acide est le N-palmitoyl glycine.

3. Procédé tel que défini à la revendication 1, pour lequel le N-acyl amino acide est le N-palmitoyl isoleucine.

## Patentansprüche

1. Verfahren zur nicht-therapeutischen Behandlung des menschlichen Körpers, die dazu vorgesehen ist, das Auftreten von Falten zu verzögern, **dadurch gekennzeichnet, dass** eine Zusammensetzung angewendet wird, die ein kosmetisch akzeptables Medium und eine wirksame Menge mindestens einer N-Acylaminosäure, die aus N-Palmitoylglycin und N-Palmitoylisoleucin ausgewählt ist, enthält.

2. Verfahren nach Anspruch 1, wobei die N-Acylaminosäure N-Palmitoylglycin ist.

3. Verfahren nach Anspruch 1, wobei die N-Acylaminosäure N-Palmitoylisoleucin ist.

## Claims

1. Method for the non-therapeutic treatment of the human body intended to delay the appearance of wrinkles, **characterised in that** a composition is applied thereto, containing a cosmetically acceptable medium and an effective quantity of at least one N-acyl amino acid chosen from N-palmitoyl glycine and N-palmitoyl isoleucine.

2. Method as defined in claim 1, for which the N-acyl amino acid is N-palmitoyl glycine.

3. Method as defined in claim 1, for which the N-acyl amino acid is N-palmitoyl isoleucine.
